# EUROPEAN PATENT SPECIFICATION

(11) **EP 1 221 913 B1**
(45) Date of publication and mention of the grant of the patent: **23.07.2008**
(21) Application number: 00973619.0
(22) Date of filing: 17.10.2000
(51) Int. Cl.: A61F 2/44

(54) **SPINAL DISC ANNULUS STENT**
STENT FÜR EINEN BANDSCHEIBENANNULUS
STENT D'ANNULUS DE DISQUE INTERVERTEBRAL

(30) Priority: 20.10.1999 US 160710 P; 18.01.2000 US 484706
(43) Date of publication of application: 17.07.2002
(73) Proprietor: Anulex Technologies, Inc., Minnetonka, MN 55343 (US)
(72) Inventor: CAUTHEN, Joseph, C., Gainesville, FL 32607 (US)
(74) Representative: Molnia, David
(86) International application number: PCT/US2000/028756
(87) International publication number: WO 2001/028464

(56) References cited:
- WO-A-94/23671
- WO-A-99/02108
- WO-A-99/16381
- WO-A1-01/10316
- DE-C- 4 323 595
- US-A- 5 634 944

## Description

### Field of the Invention

The invention generally relates to an annulus stent to be used in a surgical method of intervertebral disc wall reconstruction. The effects of said reconstruction are restoration of disc wall integrity and reduction of the failure rate (3-21%) of a common surgical procedure (disc fragment removal or discectomy). This surgical procedure is performed about 390,000 times annually in the United States.

### Background of the Invention

The spinal column is formed from a number of vertebrae, which in their normal state are separated from each other by cartilaginous intervertebral discs. The intervertebral disc acts in the spine as a crucial stabilizer, and as a mechanism for force distribution between the vertebral bodies. Without the disc, collapse of the intervertebral space occurs in conjunction with abnormal joint mechanics and premature development of arthritic changes.

The normal intervertebral disc has an outer ligamentous ring called the annulus surrounding the nucleus pulposus. The annulus binds the adjacent vertebrae together and is constituted of collagen fibers that are attached to the vertebrae and cross each other so that half of the individual fibers will tighten as the vertebrae are rotated in either direction, thus resisting twisting or torsional motion. The nucleus pulposus is constituted of loose tissue, having about 85% water content, which moves about during bending from front to back and from side to side.

As people age, the annulus tends to thicken, desicate, and become more rigid. The nucleus pulposus, in turn, becomes more viscous and less fluid and sometimes even dehydrates and contracts. The annulus also becomes susceptible to fracturing or fissuring. These fractures tend to occur all around the circumferenceof the annulus and can extend from both the outside of the annulus inwards, and the interior outward. Occasionally, a fissure from the outside of the annulus meets a fissure from the inside and results in a complete rent or tear through the annulus fibrosis. In situations like these, the nucleus pulposus may extrude out through the annulus wall. The extruded material, in turn, can impinge on the spinal cord or on the spinal nerve rootlet as it exits through the intervertebral disc foramen, resulting in a condition termed ruptured disc or herniated disc

In the event of annulus rupture, the inner-nucleus component migrates along the path of least resistance forcing the fissure to open further, allowing migration of the nucleus pulposus through the wall of the disc, with resultant nerve compression and leakage of chemicals of inflammation into the space around the adjacent nerve roots supplying the extremities, bladder, bowel and genitalia. The usual effect of nerve compression and inflammation is intolerable back or neck pain, radiating into the extremities, with accompanying numbness, weakness, and in late stages, paralysis and muscle atrophy, and/or bladder and bowel incontinence. Additionally, injury, disease or other degenerative disorders may cause one or more of the intervertebral discs to shrink, collapse, deteriorate or become displaced, herniated, or otherwise damaged.

The surgical standard of care for treatment of herniated, displaced or ruptured intervertebral discs is fragment removal and nerve decompression without a requirement to reconstruct the annular wall. While results are currently acceptable, they are not optimal. Various authors report 3.1-21% recurrent disc herniation, representing a failure of the primary procedure and requiring re-operation for the same condition. An estimated 10% recurrence rate results in 39,000 re-operations in the United States each year.

An additional method of relieving the symptoms is thermal annuloplasty, involving the heating of sub-annular zones in the non-herniated painful disc, seeking pain relief, but making no claim of reconstruction of the ruptured, discontinuous annulus wall.

Document WO 01/10316, part of the state of the art according to Article 54(3) EPC, represents the closest prior art and describes several embodiments of stents used for treating disc herniation.

There is currently no known method of annulus reconstruction, either primarily or augmented with an annulus stent according to the present invention.

### Brief Summary of the Invention

The present invention provides devices for reconstruction of the disk wall in cases of displaced, herniated, ruptured, or otherwise damaged intervertebral discs.

In some methods of annulus reconstruction, not according to the present invention, one or more mild biodegradable surgical sutures are placed at about equal distances along the sides of a pathologic aperture in the ruptured disc wall (annulus) or along the sides of a surgical incision in the weakened, thinned disc annulus.

Sutures are then tied in such fashion as to draw together the sides of the aperture, effecting reapproximation or closure of the opening, to enhance natural healing and subsequent reconstruction by natural tissue (fibroblasts) crossing the now surgically narrowed gap in the disc annulus.

A 25-30% reduction in the rate of recurrence of disc nucleus herniation through this aperture, has been achieved using this method.

In use, the method can be augmented by placement of a patch of human muscle fascia (the membrane covering the muscle) or any other autograft or allograft acting as a bridge in and across the aperture, providing a platform for traverse of fibroblasts or other normal cells of repair existing in and around the various layers of the disc annulus, prior to closure of the aperture.

A 30-50% reduction in the rate of recurrence of disc herniation has been achieved using the aforementioned fascial augmentation with this embodiment.

Having demonstrated that human muscle fascia is adaptable for annular reconstruction, other biocompatible membranes can be employed as a bridge, stent, patch or barrier to subsequent migration of the disc nucleus through the aperture. Such biocompatible materials may be, for example, a medical grade biocompatible fabric, biodegradable polymeric sheets, or form fitting or non-form fitting fillers for the cavity created by removal of a portion of the disc nucleus in the course of the disc fragment removal or discectomy. The prosthetic material can be placed in and around the intervertebral space, created by removal of the degenerated disc fragments.

### Brief Description of the Drawings

**Figure 1** shows a perspective view of the annulus stent.
**Figure 2** shows a front view of the annulus stent.
**Figure 3** shows a side view of the annulus stent.
**Figure 4A-4C** show a front view of various alternative embodiments of the annulus stent.
**Figure 5A-5B** shows the alternative embodiment of a pyramid shaped annulus stent.
**Figure 6A-6B** shows the alternative embodiment of a coned shaped annulus stent.
**Figure 7** shows the primary closure of the opening in the disc annulus, without an intervertebral or subannular stent.
**Figure 8A-8B** shows the primary closure with a stent in generic form.
**Figure 9** shows a method of suturing the annulus stent into the disc annulus, utilizing sub-annular fixation points.
**Figure 10A-10B** show the annulus stent with flexible bladder being expanded into the disc annulus.
**Figure 11A-11D** show the annulus stent being inserted into the disc annulus.
**Figure 12A-12B** show the annulus stent with the flexible bladder being expanded by injection.

### Detailed Description of the Invention

The present invention provides devices for reconstruction of the disk wall in cases of displaced, herniated, ruptured, or otherwise damaged intervertebral discs.

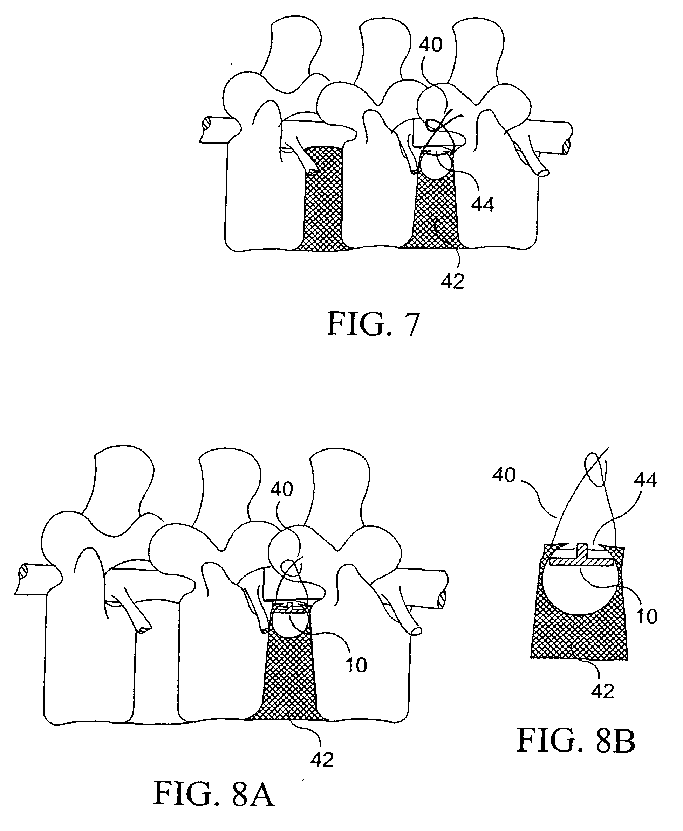

In one example, not according to the present invention, as shown in Figure 7, a damaged annulus **42** is repaired by use of surgical sutures **40.** One or more surgical sutures **40** are placed at about equal distances along the sides of a pathologic aperture **44** in the ruptured annulus **42.** Reapproximation or closure of the aperture **44** is accomplished by tying the sutures **40** in such a fashion that the sides of the aperture **44** are drawn together. The reapproximation or closure of the aperture **44** enhances the natural healing and subsequent reconstruction by the natural tissue crossing the now surgically narrowed gap in the annulus **42.** Preferably, the surgical sutures **40** are biodegradable, but permanent non-biodegradable may be utilized.

Additionally, to repair a weakened or thinned disc annulus **42,** a surgical incision is made along the weakened or thinned region of the annulus **42** and one or more surgical sutures **40** are placed at about equal distances along the sides of the incision. Reapproximation or closure of the incision is accomplished by tying the sutures **40** in such a fashion that the sides of the incision are drawn together. The reapproximationor closure of the incision enhances the natural healing and subsequent reconstruction by the natural tissue crossing the now surgically narrowed gap in the annulus **42.** Preferably, the surgical sutures **40** are biodegradable, but permanent non-biodegradable materials may be utilized.

Alternatively, the method can be augmented by the placement of a patch of human muscle fascia or any other autograft, allograft or xenograft in and across the aperture **44.** The patch acts as a bridge in and across the aperture, providing a platform for traverse of fibroblasts or other normal cells of repair existing in and around the various layers of the disc annulus, prior to closure of the aperture.

In an embodiment according to the present invention, as shown in Figure 8, a biocompatible membrane can be employed as an annulus stent **10,** being placed in and across the aperture **44.** The annulus stent **10** acts as a bridge in and across the aperture **44,** providing a platform for a traverse of fibroblasts or other normal cells of repair existing in and around the various layers of the disc annulus, prior to closure of the aperture **44.**

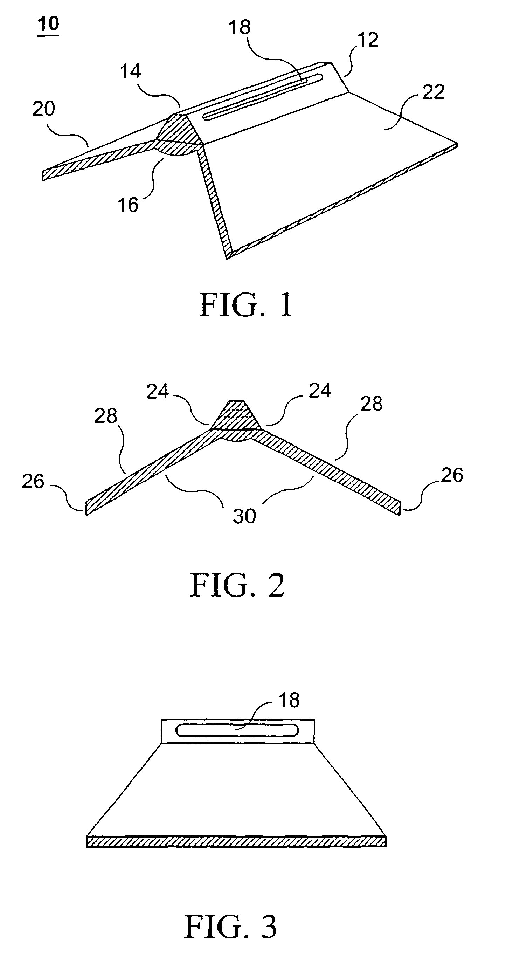

In a preferred embodiment, as shown in Figures 1-3, the annulus stent **10** comprises a centralized vertical extension **12,** with an upper section **14** and a lower section **16.** The central ized vertical extension **12** can be trapezoid in shape through the width and may be from about 8mm -12mm in length.

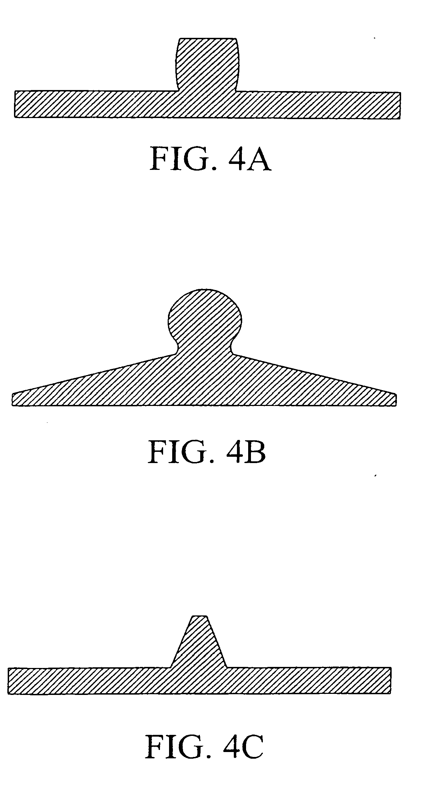

Additionally, the upper section **14** of the centralized vertical extension **12** may be any number of different shapes, as shown in Figures 4A and 4B, with the sides of the upper section **14** being curved or with the upper section **14** being circular in shape. Furthermore, the annulus stent **10** may contain a recess between the upper section **14** and the lower section **16,** enabling the annulus stent **10** to form a compatible fit with the edges of the aperture **44.**

The upper section **14** of the centralized vertical extension **12** can comprise a slot **18,** where the slot **18** forms an orifice through the upper section **14.** The slot **18** is positioned within the upper section such that **14** it traverses the upper section's **14** longitudinal axis. The slot **18** is of such a size and shape that sutures, tension bands, staples or any other type of fixation device known in the art may be passed through, to affix the annulus stent **10** to the disc annulus **44.**

In an alternative embodiment, the upper section **14** of the centralized vertical extension **12** may be perforated. The perforated upper section **14** contains a plurality of holes which traverse the upper section's **14** longitudinal axis. The perforations are of such a size and shape that sutures, tension bands, staples or any other type of fixation device known in the art may be passed through, to affix the annulus stent **10** to the disc annulus **44.**

The lower section **16** can comprise a pair of lateral extensions, a left lateral extension **20** and a right lateral extension **22.** The lateral extensions **20** and **22** comprise an inside edge **24,** an outside edge **26,** an upper surface **28,** and a lower surface **30.** The lateral extensions **20** and **22** can have an essentially constant thickness throughout. The inside edge **24** is attached to the lower section **16** and is about the same length as the lower section **16.** The outside edge **26** can be about 8mm - 16mm in length. The inside edge **24** and the lower section **16** meet to form a horizontal plane, essentially perpendicular to the centralized vertical extension **12.** The upper surface **28** of the lateral extensions **20** and **22** can form an angle of about 0°-60° below the horizontal plane. The width of the annulus stent **10** may be from about 3mm-5mm.

Additionally, the upper surface **28** of the lateral extensions **20** and **22** may be barbed for fixation to the inside surface of the disc annulus **40** and to resist expulsion through the aperture **44.**

In an alternative embodiment, as shown in Figure 4B, the lateral extensions **20** and **22** have a greater thickness at the inside edge **24** than at the outside edge **26.**

In a preferred embodiment, the annulus stent **10** is a solid unit, formed from one or more of the flexible resilient biocompatible or bioresorbable materials well know in the art.

For example, the annulus stent may be made from:
a porous matrix or mesh of biocompatible and bioresorbable fibers acting as a scaffold to regenerate disc tissue and replace annulus fibrosus as disclosed in, for example, U.S. Patent Nos. 5,108,438 (Stone) and 5,258,043 (Stone);
a strong network of inert fibers intermingled with a bioresorbable (or biosabsorable) material which attracts tissue ingrowth as disclosed in, for example, U.S. Patent No. 4,904,260 (Ray et al.);
a biodegradable substrate as disclosed in, for example, U.S. Patent No. 5,964,807 (Gan at al.); or
a expandable polytetrafluoroethylene(ePTFE), as used for conventional vascular grafts, such as those sold by W.L. Gore and Associates, Inc. under the trademarks GORE-TEX and PRECLUDE, or by Impra, Inc. under the trademark IMPRA.

Furthermore, the annulus stent **10,** may contain hygroscopic material for a controlled limited expansion of the annulus stent **10** to fill the evacuated disc space cavity.

Additionally, the annulus stent **10** may comprise materials to facilitate regeneration of disc tissue, such as bioactive silica-based materials which assist in regeneration of disc tissue as disclosed in U.S. Patent No. 5,849,331 (Ducheyne, et al.), or other tissue growth factors well known in the art.

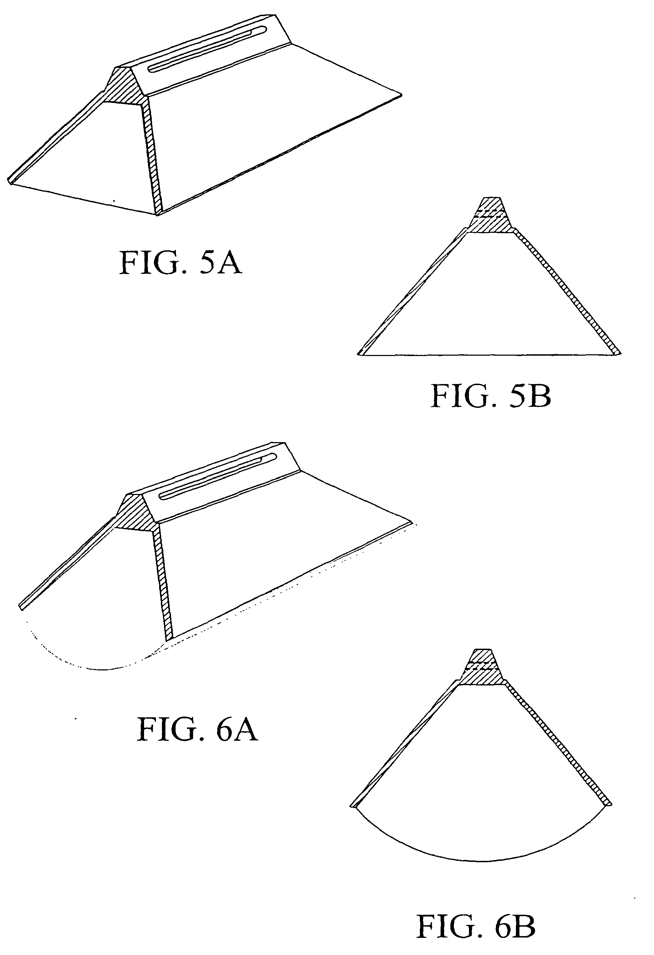

In further embodiments, as shown in Figures 5-6, the left and right lateral extensions **20** and **22** join to form a solid pyramid or cone. Additionally, the left and right lateral extensions **20** and **22** may form a solid trapezoid, wedge, or bullet shape. The solid formation may be a solid biocompatible or bioresorbableflexible material, allowing the lateral extensions **20** and **22** to be compressed for insertion into aperture **44,** then to expand conforming to the shape of the annulus' **42** inner wall.

Alternatively, a compressible core may be attached to the lower surface **30** of the lateral extensions **20** and **22,** forming a pyramid , cone, trapezoid, wedge, or bullet shape. The compressible core may be made from one of the biocompatible or bioresorbable resilient foams well known in the art. The compressible core allows the lateral extensions **20** and **22** to be compressed for insertion into aperture **44,** then to expand conforming to the shape of the annulus' **42** inner wall and to the cavity created by pathologic extrusion or surgical removal of the disc fragment.

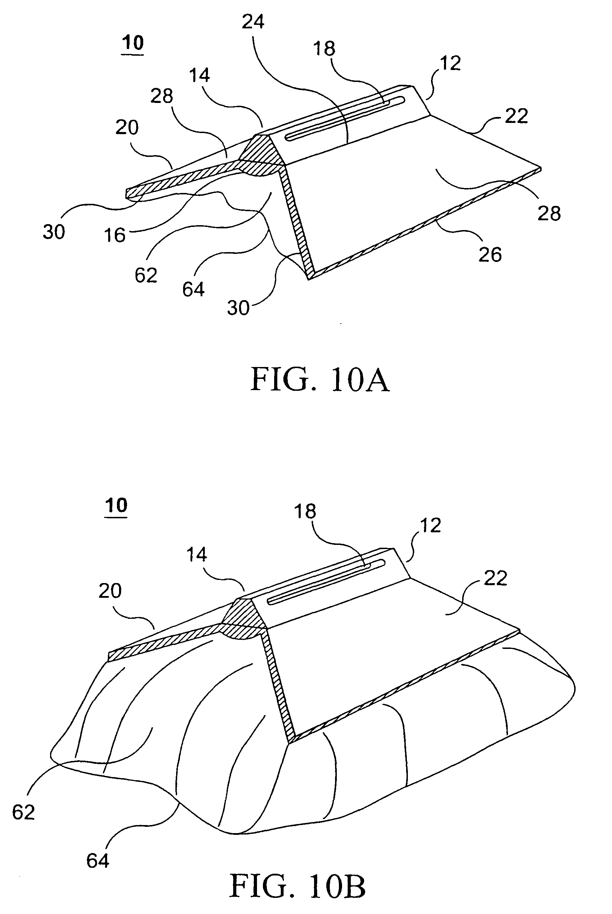

In a preferred method of use, as shown in Figures 10A-10D, the lateral extensions **20** and **22** are compressed together for insertion into the aperture **44** of the disc annulus **40.** The annulus stent **10** is then inserted into the aperture **44,** where the lateral extensions **20** and **22** expand, with the upper surface **28** contouring to the inside surface of the disc annulus **40.** The upper section **14** is positioned within the aperture **44** so that the annulus stent **10** may be secured to the disc annulus **40,** using means well known in the art.

In an alternative method, where the length of the aperture **44** is less than the length of the outside edge **26** of the annulus stent **10,** the annulus stent **10** must be inserted laterally into the aperture **44.** The lateral extensions **20** and **22** are compressed, and the annulus stent **10** is laterally inserted into the aperture **44.** The annulus stent **10** is then rotated inside the disc annulus **40,** such that the upper section **14** is pulled back through the aperture **44.** The lateral extensions **20** and **22** are then allowed to expand, with the upper surface **28** contouring to the inside surface of the disc annulus **40.** The upper section **14** is positioned within the aperture **44** such that the annulus stent **10** may be secured to the disc annulus, using means well known in the art.

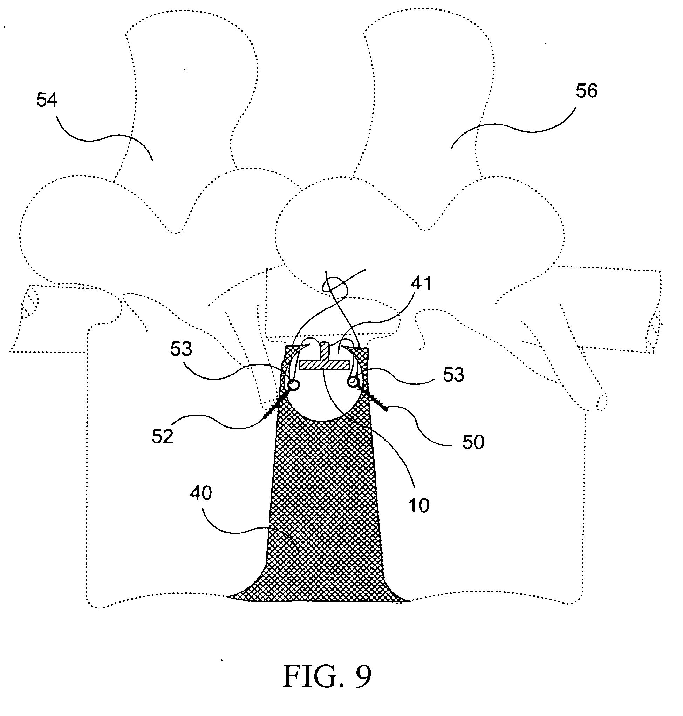

In an alternative method of securing the annulus stent **10** in the aperture **44,** as shown in Figure 9, a first surgical screw **50** and second surgical screw **52,** with eye holes **53** located at the top of the screws **50** and **52,** are opposingly inserted into the adjacent vertebrae **54** and **56** below the annulus stent **10.** After insertion of the annulus stent **10** into the aperture **44,** a suture is passed down though the disc annulus **40,** adjacent to the aperture **44,** through the eye hole **53** on the first screw **50** then back up through the disc annulus **40** and through the orifice **18** on the annulus stent **10.** This is repeated for the second screw **52,** after which the suture is secured. One or more surgical sutures **40** are placed at about equal distances along the sides of the aperture **44** in the disc annulus **42.** Reapproximation or closure of the aperture **44** is accomplished by tying the sutures **40** in such a fashion that the sides of the aperture **44** are drawn together. The reapproximation or closure of the aperture **44** enhances the natural healing and subsequent reconstruction by the natural tissue crossing the now surgically narrowed gap in the annulus **42.** Preferably, the surgical sutures **40** are biodegradable but permanent nonbiodegradable forms may be utilized. This method should decrease the strain on the disc annulus **40** adjacent to the aperture **44,** precluding the tearing of the sutures through the disc annulus **40.**

It is anticipated that fibroblasts will engage the fibers of the polymer or fabric of the intervertebral disc stent, forming a strong wall duplicating the currently existing condition of healing seen in the normal reparative process.

In an additional embodiment, as shown in Figures 10A-B, a flexible bladder **60** is attached to the lower surface **30** of the annulus stent **10.** The flexible bladder **60** comprises an internal cavity **62** surrounded by a membrane **64,** where the membrane **64** is made from a thin flexible biocompatible material. The flexible bladder **60** is attached to the lower surface **28** of the annulus stent **10** in an unexpanded condition. The flexible bladder **60** is expanded by injecting a biocompatible fluid or expansive foam, as known in the art, into the internal cavity **62.** The exact size of the flexible bladder **60** can be varied for different individuals. The typical size of an adult nucleus is 2 cm in the semi-minoraxis, 4 cm in the semi-major axis and 1.2 cm in thickness.

In an alternative embodiment, the membrane **64** is made of a semi-permeable biocompatible material.

In a preferred embodiment, a hydrogel is injected into the internal cavity of the flexible bladder **28.** A hydrogel is a substance formed when an organic polymer (natural or synthetic) is cross- linked via covalent, ionic, or hydrogen bonds to create a three-dimensional open-lattice structure which entraps water molecules to form a gel. The hydrogel may be used in either the hydrated or dehydrated form.

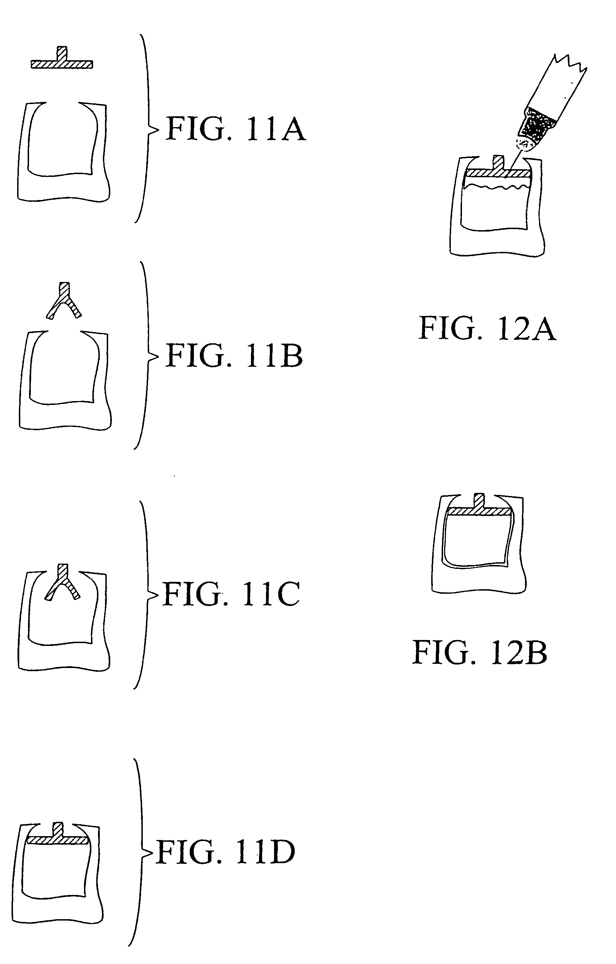

In a method of use, where the annulus stent **10** has been inserted into the aperture, as has been previously described and shown in Figures 12 A-b, an injection instrument, as known in the art, such as a syringe, is used to inject the biocompatible fluid or expansive foam into the internal cavity **62** of the flexible bladder **60.** The biocompatible fluid or expansive foam is injected through the annulus stent **10** into the internal cavity of the flexible bladder **28.** Sufficient material is injected into the internal cavity **62** to expand the flexible bladder **60** to fill the void in the intervertebral disc cavity. The use of the flexible bladder **60** is particularly useful when it is required to remove all or part of the intervertebral disc nucleus.

The surgical repair of an intervertebral disc may require the removal of the entire disc nucleus, being replaced with an implant, or the removal of a portion of the disc nucleus thereby leaving a void in the intervertebral disc cavity. The flexible bladder **60** allows for the removal of only the damaged section of the disc nucleus, with the expanded flexible bladder **60** filling the resultant void in the intervertebral disc cavity. A major advantage of the annulus stent **10** with the flexible bladder **60** is that the incision area in the annulus can be reduced in size as there is no need for the insertion of an implant into the intervertebral disc cavity.

In an alternative method of use, a dehydrated hydrogel is injected into the internal cavity **28** of the flexible bladder **60.** Fluid, from the disc nucleus, passes through the semi-permeable membrane **64** hydrating the dehydrated hydrogel. As the hydrogel absorbs the fluid the flexible bladder expands **60,** filling the void in the intervertebral disc cavity.

The patent documents U.S. Patent No. 5,108,438 (Stone), U.S. Patent No. 5,258,043 (Stone), U.S. Patent No. 4,904,260 (Ray et al.), U.S. PatentNo. 5,964,807 (Gan et al.), U.S. PatentNo. 5,849,331 (Ducheyneet al.), U.S. Patent No. 5,122,154 (Rhodes), U.S. PatentNo. 5,204,106 (Schepers at al.), U.S. Patent No. 5,888,220 (Felt et al.) and U.S. Patent No. 5,376,120 (Sarver et al.) represent prior art related to the subject-matter of the present invention.

It should be understood that the examples and embodiments described herein are for illustrative purposes only and that various modifications or changes in light thereof will be suggested to persons skilled in the art as included within the scope of the appended claims.

## Claims

1. An annulus stent for repair of an intervertebral disc annulus, comprising a centralized vertical extension (12) and left (20) and right (22) lateral extensions, suitable for being compressed for insertion into an aperture (44) of a disc annulus (42) of a patient, then for expanding to conform to the shape of the annulus' inner wall at least partially within a cavity created by pathological extrusion of surgical removal of the degenerated disc fragment, the centralized vertical extension (12) comprising an upper section (14) comprising a slot or one or more perforations (18) for receiving at least one suture, tension band, staple or any other fixation device for securing the annulus stent to the disc annulus (42) of the patient.

2. The annulus stent according to claim 1, **characterized in that** the annulus stent comprises a biocompatible membrane acting as a bridge in and across said aperture (44), or a porous matrix or mesh of biocompatible and bioresorbable fibers acting as a scaffold.

3. The annulus stent according to one of claims 1 or 2, **characterized in that** the upper section of the centralized vertical extension comprises a longitudinal axis which is traversed by the slot or perforations (18) for receiving at least one suture, tension band, staple or any other fixation device and the annulus stent is suitable for being compressed in said first configuration relative to said longitudinal axis.

4. The annulus stent according to one of claims 1 to 3, **characterized in that** the annulus stent comprises a biocompatible or bioresorbable flexible material.

5. The annulus stent according to one of claims 1 to 4, **characterized in that** the annulus stent comprises a flexible core attached to the lower surface of the lateral extensions of the annulus stent.

6. The annulus stent according to claim 5, **characterized in that** said core comprises a biocompatible or bioresorbable resilient foam.

7. The annulus stent according to one of claims 1 to 4, **characterized in that** the annulus stent comprises a flexible bladder attached to the lower surface of the lateral extensions of the annulus stent.

8. The annulus stent according to claim 7, **characterized in that** said flexible bladder comprises an internal cavity surrounded by a membrane.

9. The annulus stent according to claim 8, **characterized in that** said membrane comprises a thin flexible biocompatible material.

10. The annulus stent according to one of claims 7 to 9, **characterized in that** said flexible bladder is suitable for being expanded by injecting a biocompatible fluid.

11. The annulus stent according to claim 10, **characterized in that** said biocompatible fluid is a hydrogel.

12. The annulus stent according to claim 8, **characterized in that** said membrane comprises a semi-permeable biocompatible material.

13. The annulus stent according to claim 12, **characterized in that** said flexible bladder is suitable for being expanded by injecting a hydrogel.

14. The annulus stent according to one of the preceding claims, **characterized in that** the annulus stent comprises a biocompatible or bioresorbable flexible material selected from the group consisting of a porous matrix or mesh of biocompatible or bioresorbable fibers, inert fibers intermingled with a bioresorbable or bioabsorbable material, a biodegradeable substrate, expandable polytetrafluorethylene.

15. The annulus stent according to one of the preceding claims, **characterized in that** the annulus stent comprises a tissue growth factor.

16. The annulus stent according to one of the preceding claims, **characterized in that** the annulus stent comprises barbs for fixation to the inside surface of the disc annulus.

17. The annulus stent according to one of the preceding claims, **characterized in that** said upper section (14) comprises a slot.

18. The annulus stent according to one of claims 1 to 16, **characterized in that** said upper section (14) comprises a hole.

## Patentansprüche

1. Anulusstent zur Reparatur eines Intervertebralscheibenanulus, umfassend eine zentralisierte vertikale Erweiterung (12) sowie linke (20) und rechte (22) laterale Erweiterungen, dazu geeignet, für eine Einbringung in eine Öffnung (44) eines Scheibenanulus (42) eines Patienten komprimiert zu werden, dann zu expandieren, um sich der Form der inneren Wand des Anulus zumindest teilweise innerhalb eines durch pathologische Ausstoßung oder chirurgische Entfernung des degenerierten Scheibenfragments erzeugten Hohlraums anzupassen, wobei die zentralisierte vertikale Erweiterung (12) einen oberen Abschnitt (14) umfasst, der einen Schlitz oder eine oder mehrere Perforationen (18) zum Aufnehmen von zumindest einer Naht, einem Spannband, einer Klammer oder einer beliebigen anderen Fixierungsvorrichtung zum Sichern des Anulusstents an den Scheibenanulus (42) des Patienten umfasst.

2. Anulusstent nach Anspruch 1, **dadurch gekennzeichnet, dass** der Anulusstent eine biokompatible Membran, die als eine Brücke in und über der Öffnung (44) wirkt, oder eine poröse Matrix oder ein Netz aus biokompatiblen und bioresorbierbaren Fasern, die als ein Gerüst wirken, umfasst.

3. Anulusstent nach einem der Ansprüche 1 oder 2, **dadurch gekennzeichnet, dass** der obere Abschnitt der zentralisierten vertikalen Erweiterung eine Längsachse umfasst, die vom Schlitz oder den Perforationen (18) zum Aufnehmen von zumindest einer Naht, einem Spannband, einer Klammer oder einer beliebigen anderen Fixierungsvorrichtung durchquert wird, und dass der Anulusstent geeignet ist, in der ersten Konfiguration relativ zur Längsachse komprimiert zu werden.

4. Anulusstent nach einem der Ansprüche 1 bis 3, **dadurch gekennzeichnet, dass** der Anulusstent ein biokompatibles oder bioresorbierbares flexibles Material umfasst.

5. Anulusstent nach einem der Ansprüche 1 bis 4, **dadurch gekennzeichnet, dass** der Anulusstent einen flexiblen Kern, befestigt an die untere Oberfläche der lateralen Erweiterungen des Anulusstents, umfasst.

6. Anulusstent nach Anspruch 5, **dadurch gekennzeichnet, dass** der Kern einen biokompatiblen oder bioresorbierbaren elastischen Schaum umfasst.

7. Anulusstent nach einem der Ansprüche 1 bis 4, **dadurch gekennzeichnet, dass** der Anulusstent eine flexible Blase, befestigt an die untere Oberfläche der lateralen Erweiterungen des Anulusstents, umfasst.

8. Anulusstent nach Anspruch 7, **dadurch gekennzeichnet, dass** die flexible Blase einen inneren Hohlraum, umgeben von einer Membran, umfasst.

9. Anulusstent nach Anspruch 8, **dadurch gekennzeichnet, dass** die Membran ein dünnes flexibles biokompatibles Material umfasst.

10. Anulusstent nach einem der Ansprüche 7 bis 9, **dadurch gekennzeichnet, dass** die flexible Blase dazu geeignet ist, durch Injizieren eines biokompatiblen Fluids expandiert zu werden.

11. Anulusstent nach Anspruch 10, **dadurch gekennzeichnet, dass** das biokompatible Fluid ein Hydrogel ist.

12. Anulusstent nach Anspruch 8, **dadurch gekennzeichnet, dass** die Membran ein semipermeables biokompatibles Material umfasst.

13. Anulusstent nach Anspruch 12, **dadurch gekennzeichnet, dass** die flexible Blase dazu geeignet ist, durch Injizieren eines Hydrogels expandiert zu werden.

14. Anulusstent nach einem der vorherigen Ansprüche, **dadurch gekennzeichnet, dass** der Anulusstent ein biokompatibles oder bioresorbierbares flexibles Material, ausgewählt aus der Gruppe bestehend aus einer porösen Matrix oder einem Netz von biokompatiblen oder bioresorbierbaren Fasern, inerten Fasern, durchsetzt mit einem bioresorbierbaren oder bioabsorbierbaren Material, einem biologisch abbaubaren Substrat, expandierbarem Polytetrafluorethylen, umfasst.

15. Anulusstent nach einem der vorherigen Ansprüche, **dadurch gekennzeichnet, dass** der Anulusstent einen Gewebewachstumsfaktor umfasst.

16. Anulusstent nach einem der vorherigen Ansprüche, **dadurch gekennzeichnet, dass** der Anulusstent Widerhaken für eine Fixierung an die innere Oberfläche des Scheibenanulus umfasst.

17. Anulusstent nach einem der vorherigen Ansprüche, **dadurch gekennzeichnet, dass** der obere Abschnitt (14) einen Schlitz aufweist.

18. Anulusstent nach einem der Ansprüche 1 bis 16, **dadurch gekennzeichnet, dass** der obere Abschnitt (14) ein Loch umfasst.

## Revendications

1. Stent annulaire pour réparer un anneau de disque intervertébral, comprenant une extension verticale centrale (12) et des extensions latérales gauche (20) et droite (22), conçu pour être comprimé aux fins d'insertion dans une ouverture (44) d'un anneau de disque (42) d'un patient, puis pour s'étendre afin d'épouser la forme de la paroi interne de l'anneau au moins partiellement à l'intérieur d'une cavité créée par extrusion pathologique d'extirpation chirurgicale du fragment de disque dégénéré, l'extension verticale centrale (12) comprenant une section supérieure (14) comportant une fente ou une ou plusieurs perforations (18) pour recevoir au moins une suture, une bande de tension, une agrafe ou tout autre dispositif de fixation pour immobiliser le stent annulaire sur l'anneau du disque (42) du patient.

2. Stent annulaire selon la revendication 1, **caractérisé en ce que** le stent annulaire comprend une membrane biocompatible, agissant comme pont dans ladite ouverture (44) et à travers celle-ci, ou une matrice poreuse ou un réseau de fibres biocompatibles et biorésorbables agissant comme support.

3. Stent annulaire selon l'une de la revendication 1 ou 2, **caractérisé en ce que** la section supérieure de l'extension verticale centrale comprend un axe longitudinal qui est traversé par la fente ou des perforations (18) pour recevoir au moins une suture, une bande de tension, une agrafe ou tout autre dispositif de fixation, et **en ce que** le stent annulaire est conçu pour être comprimé dans ladite première configuration par rapport audit axe longitudinal.

4. Stent annulaire selon l'une des revendications 1 à 3, **caractérisé en ce que** le stent annulaire comprend un matériau flexible biocompatible ou biorésorbable.

5. Stent annulaire selon l'une des revendications 1 à 4, **caractérisé en ce que** le stent annulaire comprend une âme flexible attachée à la surface inférieure des extensions latérales du stent annulaire.

6. Stent annulaire selon la revendication 5, **caractérisé en ce que** la dite âme comprend une mousse résiliente biocompatible ou biorésorbable.

7. Stent annulaire selon l'une des revendications 1 à 4, **caractérisé en ce que** le stent annulaire comprend une vessie flexible attachée à la surface inférieure des extensions latérales du stent annulaire.

8. Stent annulaire selon la revendication 7, **caractérisé en ce que** la dite vessie flexible comprend une cavité interne entourée d'une membrane.

9. Stent annulaire selon la revendication 8, **caractérisé en ce que** la dite membrane comprend un matériau biocompatible flexible fin.

10. Stent annulaire selon l'une des revendications 7 à 9, **caractérisé en ce que** ladite vessie flexible est conçue pour se déployer par injection d'un fluide biocompatible.

11. Stent annulaire selon la revendication 10, **caractérisé en ce que** le fluide biocompatible est un hydrogel.

12. Stent annulaire selon la revendication 8, **caractérisé en ce que** ladite membrane comprend un matériau biocompatible semi-perméable.

13. Stent annulaire selon la revendication 12, **caractérisé en ce que** ladite vessie flexible est conçue pour se déployer par injection d'un hydrogel.

14. Stent annulaire selon l'une des revendications précédentes, **caractérisé en ce que** le stent annulaire comprend un matériau flexible biocompatible ou biorésorbable sélectionné dans le groupe consistant en une matrice poreuse ou un réseau de fibres biocompatibles ou biorésorbables, de fibres inertes entrelacées avec un matériau biorésorbable ou bioabsorbable, un substrat biodégradable, du polytétrafluoréthylène extensible.

15. Stent annulaire selon l'une des revendications précédentes, **caractérisé en ce que** le stent annulaire comprend un facteur de croissance tissulaire.

16. Stent annulaire selon l'une des revendications précédentes, **caractérisé en ce que** le stent annulaire comprend des pointes pour la fixation à la surface intérieure de l'anneau du disque.

17. Stent annulaire selon l'une des revendications précédentes, **caractérisé en ce que** la dite section supérieure (14) comprend une fente.

18. Stent annulaire selon l'une des revendications 1 à 16, **caractérisé en ce que** la dite section supérieure (14) comprend un trou.
